(19) Europäisches Patentamt
European Patent Office
Office européen des brevets

(11) **EP 3 181 159 A1**

(12) **EUROPEAN PATENT APPLICATION**

(43) Date of publication:
**21.06.2017 Bulletin 2017/25**

(51) Int Cl.:
***A61M 1/00*** *(2006.01)*

(21) Application number: **16197704.6**

(22) Date of filing: **08.11.2016**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **15.12.2015  TW 104142039**

(71) Applicant: **Apex Medical Corp.
New Taipei City, 23679 (TW)**

(72) Inventors:
• **Luo, Shih-Chao
New Taipei City, 23679 (TW)**
• **Chen, Yu-Hao
New Taipei City, 23679 (TW)**
• **Chien, Chih-Tsan
New Taipei City, 23679 (TW)**

(74) Representative: **Schwerbrock, Florian
Hagenauer Strasse 1
10435 Berlin (DE)**

(54) **PRESSURE CONTROL METHOD AND SYSTEM THEREOF**

(57)    Disclosed is a pressure control method capable of performing wound treatment by using a cover unit (12), a tube member (14, 14') and a pressure control module (16), the method comprising: (a) the pressure control module (16) forming a pressure having a first pressure value at the wound (2) via the tube member (14, 14') and the cover unit (12); (b) within a first period of time, increasing the pressure from the first pressure value to a second pressure value; (c) within a second period of time, decreasing the pressure from the second pressure value to a third pressure value; (d) after the pressure has been increased from the third pressure value to a target pressure value, the pressure control module (16) stopping increasing the pressure; (e) sampling a value from the pressure to form a sampled pressure value; and (f) according to the sampled pressure value, determining whether to perform a pressure boost process or perform step (e).

## Description

## FIELD OF THE INVENTION

The present disclosure relates to the technical field of wound treatment and more particularly to a pressure control method and a pressure control system capable of determining pressure value precisely.

## DESCRIPTION OF RELATED ART

Conventionally, hospitals used a fluid suction device, which is operated to provide negative pressure, to collect fluid exudated from patient's wound.

The fluid suction device comprises a tube and a pressure sensor. During fluid transport in the tube, because the fluid contains both gas and liquid, undesirable liquid retention in the tube results in imprecise determination of pressure in the tube by the pressure sensor, such that the fluid suction device fails to perform effective suction operation, which aggravates the wound healing process of the patient.

The fluid suction device further provides a plurality of switch buttons respectively corresponding to different fixed pressure values. According to the wound condition, particularly the fluid exudation level, of a patient, one of the switch buttons can be chosen to manage a specific fluid exudation amount with the selected pressure value. Generally, medical personnel choose one fixed pressure value within a preset time period; however, the fluid exudation amount of a wound is not always constant and is unpredictable. Therefore, conventional fluid suction devices fail to provide a proper pressure value timely and correctly, and they are therefore insufficient to properly respond to the change of fluid exudation amount.

Accordingly, the present disclosure provides a pressure control method and a system thereof to address the above-mentioned drawbacks.

## SUMMARY OF THE INVENTION

The first object of the present disclosure is to provide a pressure control method employing a tube member, a pressure control module and a cover unit to perform wound treatment on a wound, so as to remove fluid from the wound and/or supply the wound with another fluid such as medicated liquid.

The second object of the present disclosure is to provide a pressure control method capable of facilitating wound healing by adjusting the flow velocity of the fluid.

The third object of the present disclosure is to provide a pressure control method which, during the wound treatment process, changes the pressure difference between the orifices at two ends of the tube member to control the flow velocity and flow direction of the fluid, thereby directing the fluid toward the cover unit or toward the pressure control module.

The fourth object of the present disclosure is to provide a pressure control method which provides a tube member flushing mechanism and a pressure relief mechanism so as to address the problem of fluid retention in the tube member.

The fifth object of the present disclosure is to provide a pressure control method which provides a pressure supplementing mechanism so as to adjust the pressure difference between the orifices at two ends of the tube member.

The sixth object of the present disclosure is to provide a pressure control method having a continuous mode executed after the tube member flushing mechanism and the pressure relief mechanism for continuously monitoring pressure variation and, when the pressure is less than or equal to a first predetermined percentage of the absolute value of the target pressure value (e.g. 70, 100 or 125 mmHg), increasing the pressure to a level greater than the first predetermined percentage of the absolute value of the target pressure value. The continuous mode is suitable for a wound with massive amount of fluid exudate, such as fluid exudation greater than 200 mL/hr.

The seventh object of the present disclosure is to provide a pressure control method having a discrete mode executed after the tube member flushing mechanism and the pressure relief mechanism for continuously monitoring pressure variation and, when the pressure is less than or equal to the absolute value of a predetermined pressure value, increasing the pressure to the absolute value of the target pressure value. The discrete mode is suitable for a wound with small amount of fluid exudate, such as fluid exudation less than 200 mL/hr.

The eighth object of the present disclosure is to provide a pressure control method having an alarm mode, wherein the pressure control module outputs an alarm signal when the absolute value of the sampled pressure value is less than or equal to a second predetermined percentage of the absolute value of the target pressure value, the second predetermined percentage being less than the first predetermined percentage.

The ninth object of the present disclosure is to provide a pressure control system for performing wound treatment on a wound.

To achieve the above and other objects, the present disclosure provides a pressure control method for performing wound treatment on a wound by using a cover unit, a tube member and a pressure control module, the pressure control method comprising: (a) the pressure control module forming a pressure at the wound via the tube member and the cover unit, wherein the pressure has a first pressure value; (b) within a first period of time, increasing the pressure from the first pressure value to a second pressure value, wherein a pressure difference between the first pressure value and the second pressure value allows adjustment of flow velocity and flow direction of a fluid in the tube member; (c) within a second period of time, decreasing the pressure from the second pressure value to a third pressure value; (d) when the pressure is increased from the third pressure value to a target pressure value, the pressure control module stops increasing the pressure; (e) sampling a value from the pressure to form a sampled pressure value; and (f) according to the sampled pressure value, determining whether to perform a pressure boost process or perform step (e), wherein the pressure boost process comprises increasing the pressure from the sampled pressure value to the target pressure value or to be close to the target pressure value.

To achieve the above and other objects, the present disclosure provides a pressure control method for performing wound treatment on a wound by using a cover unit, a tube member and a pressure control module, the pressure control method comprising: (a') the pressure control module forming a pressure at the wound via the tube member and the cover unit, wherein the pressure has a first pressure value; (b') within a first period of time, increasing the pressure from the first pressure value to a second pressure value, wherein a pressure difference between the first pressure value and the second pressure value allows adjustment of flow velocity and flow direction of a first fluid in the tube member; (c') within a second period of time, decreasing the pressure from the second pressure value to a third pressure value; (d') injecting a second fluid into the tube member; and (e') within a third period of time, increasing the pressure from the third pressure value to a fourth pressure value, wherein a pressure difference between the fourth pressure value and the third pressure value determines flow velocity and flow direction of the second fluid in the tube member.

To achieve the above and other objects, the present disclosure provides a pressure control system for performing wound treatment on a wound, the pressure control system comprising a cover unit, a tube member and a pressure control module; the cover unit comprises a film covering the wound and an opening formed on the film; the tube member defines a first orifice and a second orifice, the first orifice being connected with the opening; the pressure control module further comprises a pressure adjustment unit, a sampling unit and a processing unit, the pressure adjustment unit and the sampling unit being coupled with the second orifice, the processing unit being connected with the pressure adjustment unit and the sampling unit, the processing unit driving the pressure adjustment unit and the sampling unit such that the pressure adjustment unit adjusts a pressure in the tube member and the sampling unit samples a value from the pressure in the tube member; wherein according to a control process, the processing unit, within a first period of time, enables the pressure adjustment unit to increase the pressure in the tube member from a first pressure value to a second pressure value such that a pressure difference between the first pressure value and the second pressure value allows adjustment of flow velocity and flow direction of a fluid in the tube member, and within a second period of time after the first period of time, enables the pressure adjustment unit to decrease the pressure from the second pressure value to a third pressure value, after which the sampling unit samples from the pressure to obtain a sampled pressure value, and the pressure adjustment unit, according to the sampled pressure value, determines whether to adjust the pressure from the sampled pressure value to a target pressure value or to be close to the target pressure value.

## BRIEF DESCRIPTION OF THE DRAWINGS

The accompanying drawings are included to provide a further understanding of the invention, and are incorporated in and constitute a part of this specification. The drawings illustrate embodiments of the disclosure and, together with the description, serve to explain the principles of the disclosure.

FIG. 1 illustrates a flowchart according to a first embodiment of the pressure control method of the present disclosure;
FIG. 2 illustrates a flowchart according to a second embodiment of the pressure control method of the present disclosure;
FIG. 3 illustrates a flowchart according to a third embodiment of the pressure control method of the present disclosure;
FIG. 4 illustrates a block diagram according to one embodiment of the pressure control system of the present disclosure;
FIG. 5(a) and FIG. 5(b) each illustrate an operational curve of the pressure control system of the present disclosure in FIG. 4;
FIG. 6 illustrates a block diagram according to one embodiment of the pressure control system of the present disclosure; and
FIG. 7 illustrates a block diagram according to another embodiment of the pressure control system of the present disclosure.

## DETAILED DESCRIPTION OF THE INVENTION

Embodiments are illustrated in the accompanying figures to improve understanding of purposes, features and effects as presented in this disclosure.

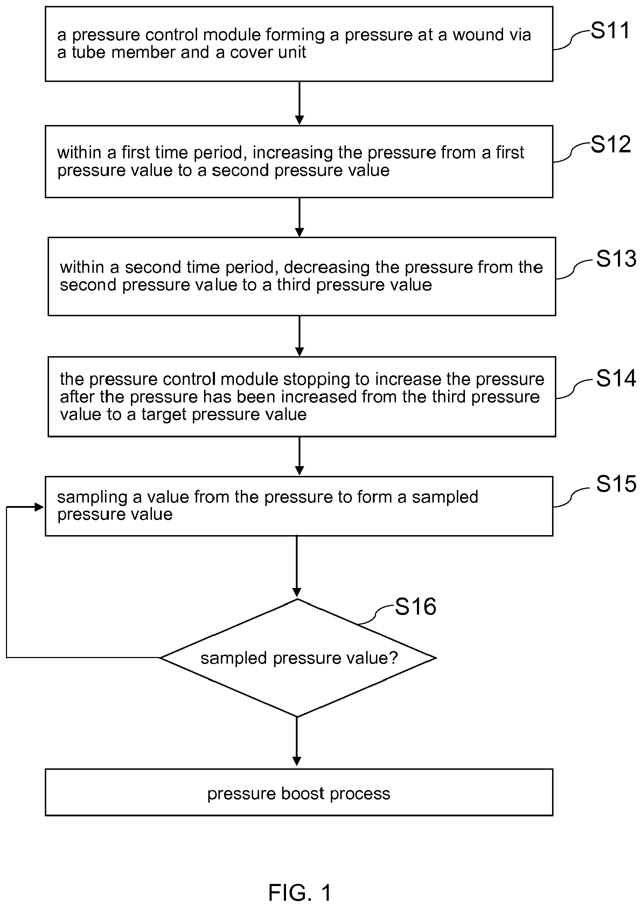

FIG. 1 illustrates a flowchart according to a first embodiment of the pressure control method of the present disclosure. As shown, the pressure control method, by using a cover unit, a tube member and a pressure control module, performs wound treatment on a wound, wherein the cover unit, the tube member and the pressure control module are described in detail below.
Before implementing the pressure control method, it is presumed that the cover unit has been covered on the wound and is protruded due to the fluid such as tissue fluid collected from the wound, thereby forming a pressure between the cover unit and the wound.

In FIG. 1, the pressure control method comprises several different mechanisms, such as a tube member flushing mechanism, a pressure relief mechanism and a pressure supplementing mechanism, which are designed as one-time operation or repetitive operations. For example, in the repetitive operations, the pressure control method is performed repetitively, wherein the duration of each pressure control method is defined as one cycle, such as one minute, several minutes or less than one minute.

In step S 11, the pressure control module forms a pressure at the wound via the tube member and the cover unit, wherein the pressure has a first pressure value. For example, when the pressure control module forms a negative pressure value at the cover unit, the pressure control module exerts a suction force on the fluid in the cover unit; when a positive pressure value is formed at the cover unit, the pressure control module exerts a pushing force on the fluid in the cover unit.

Step S12 represents an example of the aforesaid tube member flushing mechanism. In the tube member flushing mechanism, within a first time period the pressure is increased from the first pressure value to the second pressure value, and the pressure difference between the first pressure value and the second pressure value is used to adjust the flow velocity and flow direction of the fluid in the tube member; therefore, the pressure difference may regulate the flow velocity of the fluid, and the pressure difference, whether positive or negative, may determine the flow direction of the fluid.

For example, given that both the first pressure value and the second pressure value are negative values, when the pressure control module increases the pressure from the first pressure value to the second pressure value (i.e. the absolute value of the second pressure value is greater than that of the first pressure value), the pressure control module forms a suction force in the tube member, thereby directing the fluid from the cover unit toward the pressure control module and allowing the pressure control module to remove the fluid from the wound. During the process, the intensity of the suction force will change corresponding to the pressure difference. For example, the intensity of suction force formed by the second pressure value is greater than that of the first pressure value. Moreover, the first period of time can be set as several seconds, such as 10 seconds.

It should be noted that, in this step, since the pressure control module may change the flow velocity of the fluid, when the pressure control module sucks the fluid at a rate faster than the rate of fluid generation from the wound, fluid and gas concurrently exist in the tube member. Therefore, by using the tube member flushing mechanism employed in this step, the conventional problem of not being able to determine the pressure precisely due to fluid retention in the tube member can be alleviated or avoided.

Step S 13 represents an example of the aforesaid pressure relief mechanism. In the pressure relief mechanism, within a second period of time, the pressure is reduced from the second pressure value to the third pressure value.

Steps S 14 to S16 represent an example of the aforesaid pressure supplementing mechanism, which is elaborated below in detail.

In step S 14, after the pressure has been increased from the third pressure value to the target pressure value, the pressure control module stops increasing the pressure. The target pressure value may be set according to different needs; for example, the target pressure value may be generally set as 70, 100 or 124 mmHg.

In step S15, the pressure is sampled to obtain a sampled pressure value. In this step, since the pressure control module has stopped increasing the pressure, the pressure of the cover unit will therefore change corresponding to the flow rate of the fluid at the wound, and the pressure may not necessarily be maintained at the target pressure value. Therefore, this step is employed to use the sampled pressure value to determine the current pressure magnitude.

In step S16, according to the sampled pressure value, it is determined whether to perform the pressure boost process or to perform step S15. The pressure boost process refers to increasing the pressure from the sampled pressure value to the target pressure value or to be close to the target pressure value. In this step, by means of the current sampled pressure value, the pressure boost process is performed or another sampling process in step S15 is performed selectively. In step S16, the pressure boost process is further exemplified below.

The first type pressure boost process represents the aforesaid continuous mode, wherein when the absolute value of the sampled pressure value is less than or equal to a first predetermined percentage of the absolute value of the target pressure value, the pressure control module increases the pressure from the absolute value of the sampled pressure value until the pressure exceeds the first predetermined percentage (e.g. between 90% and 95%) of the absolute value of the target pressure value. After completing the pressure boost process, the pressure control module may continue to perform step S15, that is, to continuously sample the current pressure.

In addition, in this pressure boost process, the pressure control module may also directly increase the pressure from the sampled pressure value to the target pressure value or to be close to the target pressure value. Similarly, after completing the pressure boost process, the pressure control module may continue to perform step S 15.

The second type pressure boost process represents the aforesaid discrete mode, wherein when the absolute value of the sampled pressure value is greater than or equal to the absolute value of a predetermined value, which may be set as 40 mmHg for example, the pressure control module may continue to perform step S 15.

If in step S15, the sampled pressure value is determined to be less than or equal to the predetermined pressure value, the pressure control module increases the pressure from the absolute value of the sampled pressure value to the absolute value of the target pressure value.

Both the continuous mode and the discrete mode may further comprise an alarm mode. In the alarm mode, a second predetermined percentage is set in addition to the first predetermined percentage. In the alarm mode, when the absolute value of the sampled pressure value is less than or equal to the second predetermined percentage of the absolute value of the target pressure value, the pressure control module outputs an alarm signal to alarm pressure abnormality. For example, the second predetermined percentage can be set as a range of 75% to 95%. In addition, in this embodiment, the second predetermined percentage is less than the first predetermined percentage.

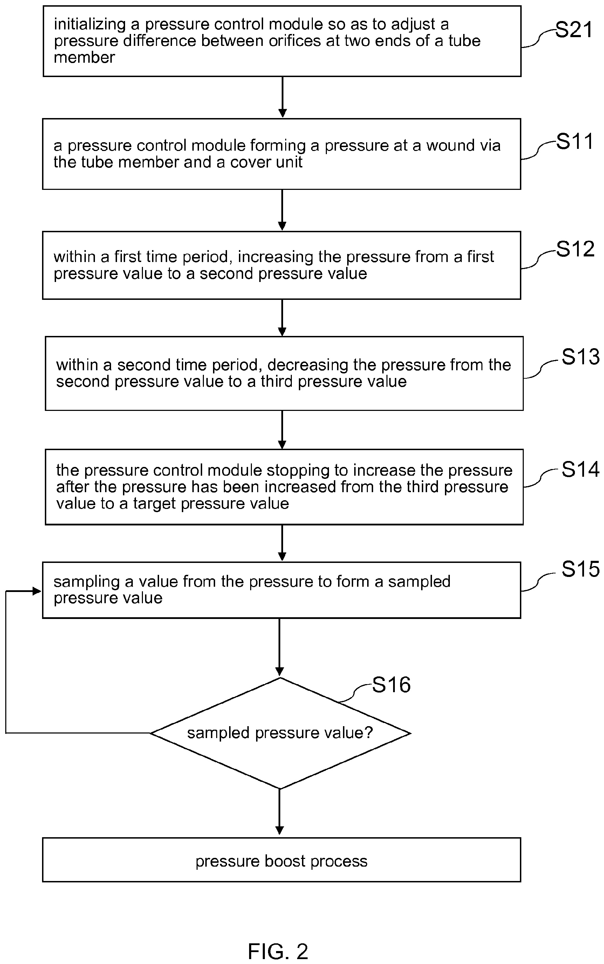

FIG. 2 illustrates a flowchart according to a second embodiment of the pressure control method of the present disclosure. As shown in FIG. 2, the pressure control method comprises, in addition to steps S11 to S16 in the first embodiment, step S21, which can be performed in any step. In this embodiment, step S21 is exemplified as being performed before step S11.

Step S21 represents an initialization process for initializing the pressure control module so as to adjust the pressure difference between the orifices at two ends of the tube member, thereby enabling the pressure control module to normally perform steps S11 to S16.

For example, the initialization process may comprise, in order, releasing the pressure in the tube member, increasing the pressure in the tube member, stopping to apply the pressure to the tube member, and stopping to release the pressure of the tube member.

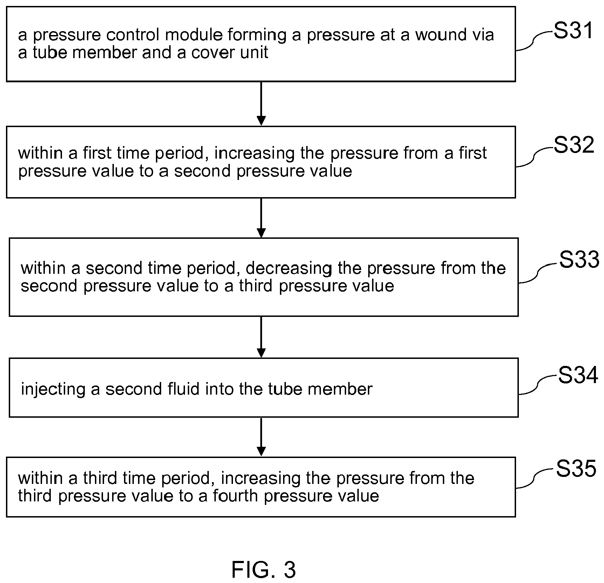

FIG. 3 illustrates a flowchart according to a third embodiment of the pressure control method of the present disclosure. As shown in FIG. 3, the pressure control method is the same as in the first embodiment, and similarly the cover unit, the tube member and the pressure control module are employed to perform wound treatment on the wound.

The pressure control method begins at step S31, which is equivalent to step S11 in the first embodiment.

Step S32 is equivalent to step S12 in the first embodiment, similarly performing the tube member flushing mechanism.

Step S33 is equivalent to step S13 in the first embodiment, similarly performing the pressure relief mechanism. For clarity and brevity, the fluid in step S13 is defined in this step as the first fluid to distinguish from the other fluid described below in other steps, which may be medicated liquid containing therapeutic ingredients for treating the wound.

In step S34, the second fluid, i.e. the aforesaid other fluid, is injected into the tube member.

In step S35, within a third period of time, the third pressure value is increased to the fourth pressure value, wherein the pressure difference between the fourth pressure value and the third pressure value is used to determine the flow velocity and flow direction of the second fluid in the tube member.

For example, if the third pressure value and the fourth pressure value are both positive values, when the pressure control module increases the pressure from the absolute value of the third pressure value to the absolute value of the fourth pressure value (i.e. the fourth pressure value is greater than the third pressure value), the pressure control module will generate a pushing force in the tube member, such that the second fluid flows from the pressure control module toward the cover unit to supply therapeutic ingredients to the wound.

It should be noted that, in addition to steps S31 to S35, an initialization step may be implemented prior to step S31, such as step S21 in the afore-mentioned second embodiment.

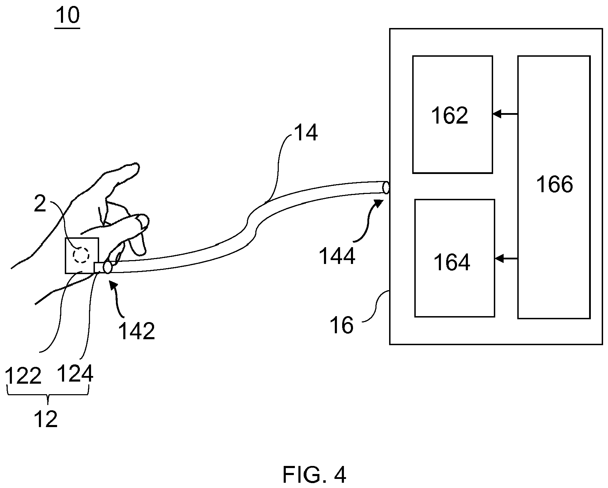

FIG. 4 illustrates a block diagram according to one embodiment of the pressure control system of the present disclosure, wherein the pressure control system 10 is capable of performing wound treatment at the wound 2.

The pressure control system 10 comprises a cover unit 12, a tube member 14 and a pressure control module 16.

The cover unit 12 comprises a film 122 covering the wound 2 and an opening 124 arranged at one side of the film 122. In this embodiment, fluid exudated from the wound 2 is concentrated and confined in the film 122 and may only leak from the opening 124.

The tube member 14 defines a first orifice 142 and a second orifice 144. The tube member 14 serves as a vehicle or carrier allowing the fluid to flow between the cover unit 12 and the pressure control module 16. In this embodiment, the tube member 14 is exemplified as one circular tube; however, in other embodiments, the shape and amount of the tube member 14 are not particularly limited. The first orifice 142 is connected with the opening 124, and the second orifice 144 is connected with the pressure control module 16.

The pressure control module 16 comprises a pressure adjustment unit 162, a sampling unit 164 and a processing unit 166. The pressure adjustment unit 162 and the sampling unit 164 are coupled with the second orifice 144. The processing unit 166 is connected with the pressure adjustment unit 162 and the sampling unit 164. The processing unit 166 drives the pressure adjustment unit 162 and the sampling unit 164, such that the pressure adjustment unit 162 adjusts the pressure in the tube member 14, and the sampling unit 164 samples a value from the pressure in the tube member 14. For example, the pressure adjustment unit 162 may comprise a servomotor, an air valve and a manifold, which collectively introduce gas employed by the pressure adjustment unit 162 to adjust the flow velocity and flow direction of the fluid in the tube member 14. The sampling unit 164 is a pressure sensor capable of detecting the pressure in the tube member 14.

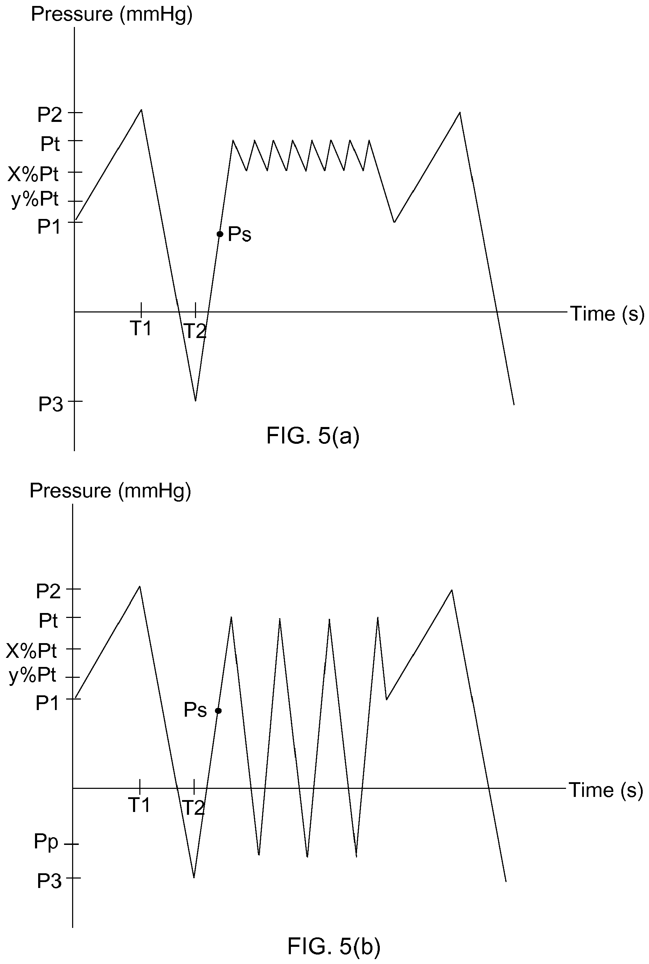

Furthermore, the processing unit 166 may execute a control process to drive the pressure adjustment unit 162 and the sampling unit 164. For example, refer to both FIG. 5(a) and FIG. 5(b), which illustrate operational curves of the pressure control system 10 in FIG. 4, wherein the vertical axis represents pressure value (mmHg), and the horizontal axis represents time (second).

As shown in FIG. 5(a) and FIG. 5(b), during the execution of the control process, in the first duration T1, the pressure adjustment unit 162 increases the pressure in the tube member 14 from the first pressure value P1 to the second pressure value P2. In the second duration T2 following the first duration T1, the pressure adjustment unit 162 decreases the pressure from the second pressure value P2 to the third pressure value P3. Subsequently, after the second duration T2, the sampling unit 164 samples from the pressure to obtain at least one sampled pressure value $Ps$. For brevity, only one sampled pressure value $Ps$ is exemplified in FIG. 5(a) and FIG. 5(b).

The pressure adjustment unit 162, based on the sampled pressure value $Ps$, may further determine whether to adjust the pressure from the sampled pressure value $Ps$ to the target pressure value $Pt$ or to be close to the target pressure value $Pt$ according to at least one of the following formula criteria or conditions.

The first formula represents the aforesaid continuous mode, which may be comprehended in conjunction with the curve in FIG. 5(a). In the continuous mode, when the sampled pressure value $Ps$ is less than the first percentage (coefficient $x$) of the target pressure value $Pt$, the pressure is adjusted from the sampled pressure value $Ps$ to the target pressure value $Pt$ or to be close to the target pressure value $Pt$, such as Formula 1.1; in contrast, if the sampled pressure value $Ps$ is greater than the first percentage of the target pressure value $Pt$, then the pressure adjustment unit 162 will not adjust the pressure, such as Formula 1.2.

$$|Ps| \leq x\% \times |Pt| \text{ (Formula 1.1)}$$

$$|Ps| \geq x\% \times |Pt| \text{ (Formula 1.2)}$$

The second formula represents the aforesaid discrete mode, which may be comprehended in conjunction with the curve in FIG. 5(b). In the discrete mode, when the sampled pressure value $Ps$ is less than the predetermined pressure value $Pp$, the pressure is adjusted from the sampled pressure value $Ps$ to the target pressure value $Pt$ or to be close to the target pressure value $Pt$, such as Formula 2.1; in contrast, if the sampled pressure value $Ps$ is greater than the predetermined pressure value $Pp$, then the pressure adjustment unit 162 will not adjust the pressure, such as Formula 2.2.

$$|Ps| \leq |Pp| \text{ (Formula 2.1)}$$

$$|Ps| \geq |Pp| \text{ (Formula 2.2)}$$

In addition, either in the continuous mode or in the discrete mode, another formula criterion or condition, corresponding to the alarm mode mentioned above, may be added for determining whether the sampled pressure value $Ps$ is less than or equal to the second percentage (coefficient $y$) of the target pressure value $Pt$. When the condition of the alarm mode is met, the pressure control module 162 outputs an alarm signal. In this embodiment, the second percentage coefficient $y$ is less than the first percentage coefficient $x$.

$$|Ps| \leq y\% \times |Pt|, y < x$$

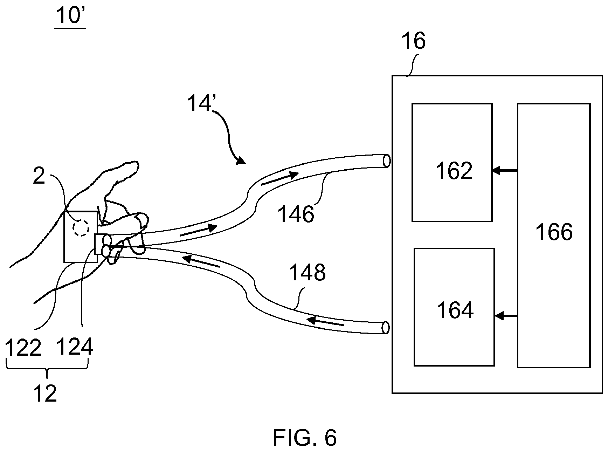

FIG. 6 illustrates a block diagram according to one embodiment of the pressure control system of the present disclosure, wherein the pressure control system 10' comprises the cover unit 12 and the pressure control module 16 illustrated in FIG. 4; unlike the previous embodiment, FIG. 6 illustrates a plurality of tube members 14'.

In this embodiment, the cover unit 12 and the pressure control module 16 are identical to those described in the first embodiment.

The tube members 14' are composed by and designated as the first tube member 146 and the second tube member 148. The pressure control module 16 forms a suction force in the first tube member 146 and a pushing force in the second tube member 148. Therefore, the pressure control module 16 may withdraw or suck the fluid from the cover unit 12 via the first tube member 146, and the pressure control module 16 may deliver the medicated fluid via the second tube member 148 to the cover unit 12.

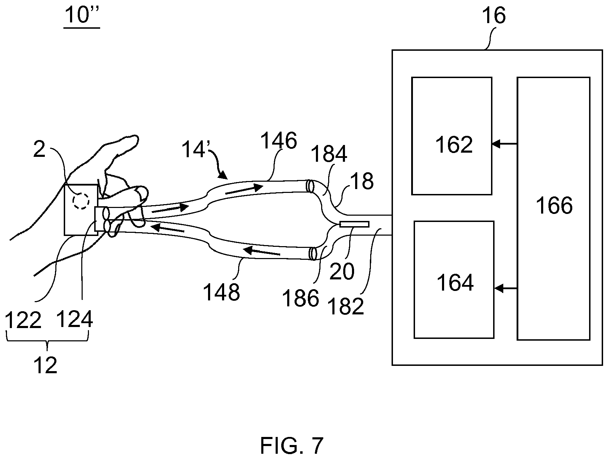

FIG. 7 illustrates a block diagram according to another embodiment of the pressure control system of the present disclosure, wherein the pressure control system 10" further comprises a manifold 18 and a regulating valve 20 in addition to the cover unit 12, the tube member 14' and the pressure control module 16 as in the previous embodiment.

The manifold 18 forms a plurality of channels 182, 184, 186. In this embodiment, the manifold 18 is shown as a Y-shape configuration by example. The channel 182 is connected with the pressure control module 16, the channel 184 is connected with the first tube member 146, and the channel 186 is connected with the second tube member 148.

The regulating valve 20 is arranged among the channels 182, 184, 186, and it may, by means of time-division multiplexing, form the suction force or the pushing force at the cover unit 12. During the time-division multiplexing operation, the regulating valve 20 forms either the suction force or the pushing force, but not both, at the cover unit 12 within a time period, thereby advantageously allowing the reduction of the number and costs of the pressure control module 16.

While preferred exemplary embodiments have been presented in the foregoing detailed description, it should be appreciated that the exemplary one or more embodiments described herein are not intended to limit the scope, applicability, or configuration of the claimed subject matter in any way. Various changes can be made in the function and arrangement of elements without departing from the scope defined by the claims, which include known equivalents and foreseeable equivalents at the time of filing this patent application.

**Claims**

1.   A pressure control method for performing wound treatment on a wound (2) by using a cover unit (12), a tube member (14, 14') and a pressure control module (16), the pressure control method being **characterized by** comprising:

(a) the pressure control module (16) forming a pressure at the wound (2) via the tube member (14, 14') and the cover unit (12), wherein the pressure has a first pressure value;
(b) within a first period of time, increasing the pressure from the first pressure value to a second pressure value, wherein a pressure difference between the first pressure value and the second pressure value allows adjustment of flow velocity and flow direction of a fluid in the tube member (14, 14'), and wherein the fluid in the tube member (14, 14') flows from the cover unit (12) toward the pressure control module (16);
(c) within a second period of time, decreasing the pressure from the second pressure value to a third pressure value;
(d) when the pressure is increased from the third pressure value to a target pressure value, the pressure control module (16) stops increasing the pressure;
(e) sampling a value from the pressure to form a sampled pressure value; and
(f) according to the sampled pressure value, determining whether to perform a pressure boost process or perform step (e), wherein the pressure boost process comprises increasing the pressure from the sampled pressure value to the target pressure value or to be close to the target pressure value.

2.   The pressure control method of claim 1, wherein the pressure boost process in step (f) comprises, when an absolute value of the sampled pressure value is not greater than a first predetermined percentage of an absolute value of the target pressure value, the pressure control module (16) increasing the pressure from the sampled pressure value to the target pressure value or increasing the pressure from the absolute value of the sampled pressure value to a level greater than the first predetermined percentage of the absolute value of the target pressure value, wherein the pressure control module (16) performs step (e) when the absolute value of the sampled pressure value is greater than the first predetermined percentage of the absolute value of the target pressure value.

3.   The pressure control method of claim 2, wherein the pressure boost process in step (f) further comprises, when the absolute value of the sampled pressure value is not greater than a second predetermined percentage of the absolute value of the target pressure value, the pressure control module (16) outputting an alarm signal, wherein the second predetermined percentage is less than the first predetermined percentage.

4.   The pressure control method of claim 1, wherein the pressure control module (16) performs step (e) when the absolute value of the sampled pressure value is greater than an absolute value of a predetermined pressure value.

5.   The pressure control method of claim 4, wherein the pressure boost process in step (f) comprises, when the absolute value of the sampled pressure value is not greater than the absolute value of the predetermined pressure value, the pressure control module (16) increasing the pressure from the absolute value of the sampled pressure value to the absolute value of the target pressure value.

6.   The pressure control method of claim 5, wherein the pressure boost process in step (f) comprises, when the absolute value of the sampled pressure value is not greater than a second predetermined percentage of the absolute value of the target pressure value, the pressure control module (16) outputting an alarm signal.

7.   The pressure control method of claim 1, further comprising, before step (a), step (g) for initializing the pressure control module (16) to adjust a pressure difference between orifices at two ends of the tube member (14, 14') so as to enable the pressure control module (16) to normally perform steps (a) to (f).

8.   The pressure control method of claim 1, further comprising after step (c):

(d') injecting a second fluid into the tube member (14, 14'); and

(e') within a third period of time, increasing the pressure from the third pressure value to a fourth pressure value, wherein a pressure difference between the fourth pressure value and the third pressure value determines flow velocity and flow direction of the second fluid in the tube member (14, 14'), and wherein the second fluid in the tube member (14,14') flows from the pressure control module (16) toward the cover unit (12).

9. A pressure control system (10, 10', 10") for performing wound treatment on a wound (2), the pressure control system (10, 10', 10") being **characterized by** comprising:

a cover unit (12) comprising a film (122) covering the wound (2) and an opening (124) formed on the film (122);

a tube member (14, 14') defining a first orifice (142) and a second orifice (144), the first orifice (142) being connected with the opening (124); and

a pressure control module (16) comprising a pressure adjustment unit (162), a sampling unit (164) and a processing unit (166), the pressure adjustment unit (162) and the sampling unit (164) being coupled with the second orifice (144), the processing unit (166) being connected with the pressure adjustment unit (162) and the sampling unit (164), the processing unit (166) driving the pressure adjustment unit (162) and the sampling unit (164) such that the pressure adjustment unit (162) adjusts a pressure in the tube member (14, 14') and the sampling unit (164) samples a value from the pressure in the tube member (14, 14');

wherein according to a control process, the processing unit (166), within a first period of time, enables the pressure adjustment unit (162) to increase the pressure in the tube member (14, 14') from a first pressure value to a second pressure value such that a pressure difference between the first pressure value and the second pressure value allows adjustment of flow velocity and flow direction of a fluid in the tube member (14, 14'), and within a second period of time after the first period of time, enables the pressure adjustment unit (162) to decrease the pressure from the second pressure value to a third pressure value, after which the sampling unit (164) samples from the pressure a sampled pressure value, and the pressure adjustment unit (162), according to the sampled pressure value, determines whether to adjust the pressure from the sampled pressure value to a target pressure value or to be close to the target pressure value.

10. The pressure control system (10, 10', 10") of claim 9, which comprises a plurality of tube members (14, 14'), and the pressure control module (16) exerts a suction force on one of the tube members (14, 14') and exerts a pushing force on the other of the tube members (14, 14').

11. The pressure control system (10, 10', 10") of claim 10, further comprising a manifold (18) defining a plurality of channels (182, 184, 186) respectively connected with the pressure control module (16) and the tube members (14, 14').

12. The pressure control system (10, 10', 10") of claim 11, further comprising a regulating valve (20) arranged between the channels (182, 184, 186) to create the suction force or the pushing force with respect to the cover unit (12) within a period of time by means of time-division multiplexing.

13. The pressure control system (10, 10', 10") of claim 9, wherein when the sampled pressure value meets the following conditions, the pressure adjustment unit (162) adjusts the pressure from the sampled pressure value to the target pressure value or to be close to the target pressure value;

$$|Ps| \leq x\% \times |Pt|;$$

or

$$|Ps| \leq |Pp|$$

wherein $Ps$ represents the sampled pressure value, $x$ represents a first percentage coefficient, $Pt$ represents the target pressure value, and $Pp$ represents a predetermined pressure value.

14. The pressure control system (10, 10', 10") of claim 13, wherein when the sampled pressure value meets the following conditions, the pressure adjustment unit (162) does not adjust the pressure;

$$|Ps| \geq x\% \times |Pt|;$$

or

$$|Ps| \geq |Pp|$$

15. The pressure control system (10, 10', 10") of claim 13, wherein when the sampled pressure value meets the following conditions, the pressure control module (16) outputs an alarm signal;

$$|Ps| \leq y\% \times |Pt|, \mathbf{y} < \mathbf{x};$$

wherein *y* represents a second percentage coefficient.

| a pressure control module forming a pressure at a wound via a tube member and a cover unit | S11 |

| within a first time period, increasing the pressure from a first pressure value to a second pressure value | S12 |

| within a second time period, decreasing the pressure from the second pressure value to a third pressure value | S13 |

| the pressure control module stopping to increase the pressure after the pressure has been increased from the third pressure value to a target pressure value | S14 |

| sampling a value from the pressure to form a sampled pressure value | S15 |

S16

sampled pressure value?

pressure boost process

FIG. 1

initializing a pressure control module so as to adjust a pressure difference between orifices at two ends of a tube member — S21

a pressure control module forming a pressure at a wound via the tube member and a cover unit — S11

within a first time period, increasing the pressure from a first pressure value to a second pressure value — S12

within a second time period, decreasing the pressure from the second pressure value to a third pressure value — S13

the pressure control module stopping to increase the pressure after the pressure has been increased from the third pressure value to a target pressure value — S14

sampling a value from the pressure to form a sampled pressure value — S15

sampled pressure value? — S16

pressure boost process

FIG. 2

| a pressure control module forming a pressure at a wound via a tube member and a cover unit | S31 |
| within a first time period, increasing the pressure from a first pressure value to a second pressure value | S32 |
| within a second time period, decreasing the pressure from the second pressure value to a third pressure value | S33 |
| injecting a second fluid into the tube member | S34 |
| within a third time period, increasing the pressure from the third pressure value to a fourth pressure value | S35 |

FIG. 3

FIG. 4

FIG. 5(a)

FIG. 5(b)

10'

16

14'

2

162

166

146

164

148

122   124

12

FIG. 6

10''

16

2

14'

146

184

18

162

166

148

20

164

186  182

122  124

12

FIG. 7

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/015585 A1 (SVEDMAN PAL [SE] ET AL) 20 January 2011 (2011-01-20) * paragraphs [0021] - [0038], [0052]; claims 11,30; figures 3-7 * | 1-15 | INV. A61M1/00 |
| X | US 2003/040687 A1 (BOYNTON THOMAS A [US] ET AL) 27 February 2003 (2003-02-27) * paragraphs [0044] - [0045]; figure 5 * | 1-15 | |
| X | WO 2009/089390 A2 (BLUESKY MEDICAL GROUP INC [US]; SMITH & NEPHEW [GB]; WESTON RICHARD SC) 16 July 2009 (2009-07-16) * paragraph [0092]; figures 7E, 7G * | 1-15 | |

TECHNICAL FIELDS SEARCHED (IPC)

A61M

The present search report has been drawn up for all claims

| Place of search | Date of completion of the search | Examiner |
|---|---|---|
| The Hague | 21 April 2017 | Westsson, David |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 16 19 7704

This annex lists the patent family members relating to the patent documents cited in the above-mentioned European search report.
The members are as contained in the European Patent Office EDP file on
The European Patent Office is in no way liable for these particulars which are merely given for the purpose of information.

21-04-2017

| Patent document cited in search report | | | Publication date | Patent family member(s) | | | Publication date |
|---|---|---|---|---|---|---|---|
| US 2011015585 | A1 | | 20-01-2011 | US | 2011015585 | A1 | 20-01-2011 |
| | | | | US | 2011112494 | A1 | 12-05-2011 |
| US 2003040687 | A1 | | 27-02-2003 | AT | 424863 | T | 15-03-2009 |
| | | | | AT | 482730 | T | 15-10-2010 |
| | | | | AU | 2002329844 | B2 | 22-05-2008 |
| | | | | AU | 2008202973 | A1 | 31-07-2008 |
| | | | | AU | 2011201729 | A1 | 12-05-2011 |
| | | | | AU | 2011201730 | A1 | 19-05-2011 |
| | | | | BR | 0212058 | A | 17-08-2004 |
| | | | | CA | 2458285 | A1 | 06-03-2003 |
| | | | | CN | 1571682 | A | 26-01-2005 |
| | | | | CN | 1973916 | A | 06-06-2007 |
| | | | | CN | 101791443 | A | 04-08-2010 |
| | | | | CY | 1109138 | T1 | 02-07-2014 |
| | | | | DE | 07117289 | T1 | 31-07-2008 |
| | | | | DE | 07117293 | T1 | 31-07-2008 |
| | | | | DK | 1418973 | T3 | 24-01-2011 |
| | | | | DK | 1897569 | T3 | 29-06-2009 |
| | | | | EP | 1418973 | A2 | 19-05-2004 |
| | | | | EP | 1897569 | A1 | 12-03-2008 |
| | | | | EP | 1900383 | A1 | 19-03-2008 |
| | | | | EP | 2052750 | A1 | 29-04-2009 |
| | | | | ES | 2299412 | T1 | 01-06-2008 |
| | | | | ES | 2299413 | T1 | 01-06-2008 |
| | | | | ES | 2353863 | T3 | 07-03-2011 |
| | | | | HK | 1061982 | A1 | 04-11-2011 |
| | | | | HK | 1105175 | A1 | 31-12-2010 |
| | | | | IL | 160450 | A | 15-05-2007 |
| | | | | IL | 181087 | A | 30-11-2010 |
| | | | | JP | 4667497 | B2 | 13-04-2011 |
| | | | | JP | 4709299 | B2 | 22-06-2011 |
| | | | | JP | 4709302 | B2 | 22-06-2011 |
| | | | | JP | 5242663 | B2 | 24-07-2013 |
| | | | | JP | 5628773 | B2 | 19-11-2014 |
| | | | | JP | 2005500141 | A | 06-01-2005 |
| | | | | JP | 2009056335 | A | 19-03-2009 |
| | | | | JP | 2009233371 | A | 15-10-2009 |
| | | | | JP | 2009254887 | A | 05-11-2009 |
| | | | | JP | 2011031089 | A | 17-02-2011 |
| | | | | JP | 2012011261 | A | 19-01-2012 |
| | | | | KR | 20040039301 | A | 10-05-2004 |
| | | | | KR | 20070072625 | A | 04-07-2007 |
| | | | | KR | 20080066874 | A | 16-07-2008 |
| | | | | KR | 20090095605 | A | 09-09-2009 |
| | | | | MX | PA04001457 | A | 13-09-2004 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 16 19 7704

This annex lists the patent family members relating to the patent documents cited in the above-mentioned European search report.
The members are as contained in the European Patent Office EDP file on
The European Patent Office is in no way liable for these particulars which are merely given for the purpose of information.

21-04-2017

| Patent document cited in search report | Publication date | Patent family member(s) | | Publication date |
|---|---|---|---|---|
| | | NZ | 531268 A | 29-09-2006 |
| | | PT | 1418973 E | 31-12-2010 |
| | | PT | 1897569 E | 17-06-2009 |
| | | RU | 2302263 C2 | 10-07-2007 |
| | | RU | 2370285 C2 | 20-10-2009 |
| | | US | 2003040687 A1 | 27-02-2003 |
| | | US | 2006149170 A1 | 06-07-2006 |
| | | US | 2011022013 A1 | 27-01-2011 |
| | | US | 2014257212 A1 | 11-09-2014 |
| | | US | 2016235897 A1 | 18-08-2016 |
| | | WO | 03018098 A2 | 06-03-2003 |
| | | ZA | 200401261 B | 27-07-2005 |
| WO 2009089390 A2 | 16-07-2009 | AT | 546174 T | 15-03-2012 |
| | | AU | 2009204140 A1 | 16-07-2009 |
| | | CA | 2711620 A1 | 16-07-2009 |
| | | DK | 2242522 T3 | 18-06-2012 |
| | | EP | 2242522 A2 | 27-10-2010 |
| | | EP | 2452704 A1 | 16-05-2012 |
| | | ES | 2382595 T3 | 11-06-2012 |
| | | JP | 5645669 B2 | 24-12-2014 |
| | | JP | 2011509160 A | 24-03-2011 |
| | | US | 2010298792 A1 | 25-11-2010 |
| | | US | 2013144233 A1 | 06-06-2013 |
| | | US | 2016144082 A1 | 26-05-2016 |
| | | WO | 2009089390 A2 | 16-07-2009 |

EPO FORM P0459

For more details about this annex : see Official Journal of the European Patent Office, No. 12/82

page 2 of 2